# EUROPEAN PATENT APPLICATION

(11) **EP 2 653 156 A1**
(43) Date of publication of application: **23.10.2013**
(21) Application number: 12186840.0
(22) Date of filing: 01.10.2012
(51) Int. Cl.: A61K 9/10, A61K 9/107, A61K 9/14, A61K 38/28, A61K 9/51, A61P 3/10

(54) **Pharmaceutical emulsion composition and use thereof**

(71) Applicant: College of Pharmacy of Taif University, 21944 Taif (SA)
(72) Inventor: Al-Remawi, Mayyas, 21944 Taif (SA); Maghrabi, Ibrahim, 21944 Taif (SA)
(74) Representative: Scholz, Volker

(57) **Abstract**

The present invention relates to a pharmaceutical emulsion composition comprising: at least one drug, at least one amino acid, at least one fatty acid and at least one surfactant, wherein the amino acid, the fatty acid and the surfactant form micelles and the drug is encapsulated into the micelles and its use as a medicament, in particular for oral administration in treatment of hyperglycemia and diabetes.

## Description

The present invention relates to a pharmaceutical emulsion composition and its use as a medicament for oral administration, in particular for use in treatment of hyperglycemia and diabetes.

The preparation of a novel nanoparticle system that is able to deliver proteins via oral route is considered an important goal for delivery systems based on nanotechnology. This will enable patients to take their protein medicaments through oral route instead of injectables.

The development of nanoparticles for oral delivery of proteins has been described widely in literature. Nanoparticles could be prepared by the use of polar lipids acting as emulsifying agents to form micellar or liposomal or nanovesicle systems. The use of polar lipid structures within the nanoparticle systems resulted in pronounced enhancement in protein drug permeation and hence increases protein oral bioavailability. The polar lipids were previously prepared through chemical conjugation of the lipids with polar structures such as amino acids forming lipoamino acids.

The lipoamino acids are present naturally in cell membrane of many organisms. Under certain stress conditions specific membrane lipids can be formed. For example, under phosphorus limiting conditions of growth, some bacteria form membrane lipids lacking phosphorus such as betaine lipids or ornithine-containing lipids. The lipoamino acids could be either simple or complex in its structure. The simple forms of those lipoamino acids contain only amino acid and fatty acid(s). They are present exclusively in bacteria and lower plants (fern, algae, protozoa). There are two groups of complex lipoamino acids: (a) lipids having an amino acid with N-acyl and/or ester link such as lysine-containing lipids (found in Streptomyces species of bacteria) or Ornithine-containing lipids (in photosynthetic purple bacteria) and (b) lipids having a glycerol and an amino acid with ether link. These lipids are glycerolipids and are mainly derived from homoserine, they are characteristic of algae.

Nevertheless, in order to form lipid bilayer membranes, lipids usually need to have long-chain acyl or alkyl residues and the molecules should be roughly of cylindric shape connected to amphiphilic heads of aminoacids. However, it is important to mention that many of lipoamino acids are present in bacteria as bioactive molecules. Many were acting as antibiotics and biosurfactants. Thus, formulation of lipoamino acids as carriers for drug delivery should be taken with caution.

The conjugation of amino acids with short lipid moieties in form of N-acetyl conjugate with the 20 amino acids is also common in mammals. However, conjugation with long chain fatty acids in form of N-acyl conjugate is less common in mammals. The most common mammalian N-fatty acylamino acids are conjugates of glycine, glutamine and taurine. One purpose of N-acetyl or N-acyl conjugation would be detoxification and elimination. It is believed that the N-acyl amino acids would have a specific pharmacological or metabolic role in mammals. For example, N-arachidonoyl-L-serine was isolated from bovine brain and showed that material had vasodilatory properties. Other examples of N-acyl amino acids that possess pharmacological actions are N-oleoylglycine regulates body temperature and locomotion, N-arachidonoyltaurine activates TRPV1 and TRPV4 calcium channels of the kidney, N-arachidonoylglycine is an endogenous ligand for the orphan GPR18 receptor, N-arachidonoyl-g-aminobutyric acid and N-arachidonoylglycine are analgesics.

Another set of N-acyl conjugates found in eukaryotes is myristic acid, lauric acid, (cis-D5)-tetradecaenoic acid (physeteric acid), (cis,- cis-D5,D8)-tetradecadienoyl, and palmitic acid, have been identified as N-terminal fatty acids of the amino acids.

Due to their specific polar lipidic structures, it is well-known that lipoaminoacids and lipopolyaminoacids can easily go through the tissues and cellular membranes. Therefore, many synthetic lipoamino acids were prepared through chemical conjugation and used extensively as drug carriers. The inclusion of lipoamino acids with gene delivery carriers has shown positive results for the delivery of an oligonucleotide and they have also shown the ability to protect DNA against nucleases.

Chemical conjugation of protein drugs with lipoamino acids was demonstrated to increase protein drug permeability. Thyrotropin releasing hormone (TRH) and luteinizing hormone releasing hormone (LHRH) were conjugated with two lipoamino acids. The conjugates developed have been absorbed and detected after oral administration and appear to be stable for a considerable time in vivo. Lipoamino acids have been also used in conjugation with liposome in many previous studies. Due to the presence of many naturally occurring lipoamino acids that act as body regulators and having specific metabolic function, one cannot ignore the fact that the introduction of a new synthetic lipoamino acid could interfere or result into unknown pharmacologic or toxicologic action either through the direct action of the parent lipoamino acid molecule or one of its metabolites. The challenge of measuring toxicity of a new synthetic lipoamino acid could limit their use as drug carriers in the pharmaceutical industry due to the regulatory restriction.

Insulin is usually secreted in response to the increase in blood sugar so its secretion will result in a balance in the blood glucose level a long with the action of other hormones. The body's failure to produce insulin resulted in a disease called type 1 diabetes, and presently requires the patients to inject themselves with insulin preparations.

Previous studies using physically modified lipo-polysaccharides forming nanoparticles such as chitosan resulted in pronounced activity for insulin when given orally (Badwan et al. Neuroendocrinology Letters, 2009, 30(1); Elsayed et al. European Journal of Pharmaceutics and Biopharmaceutics, 2009,73(2), 269-279; EP 2 042 166; CA 2 672 707).

It is an objection of the present invention to provide a carrier system for drugs, in particular for protein drugs such as insulin, allowing transport of the drug through the harsh environment of the gastrointestinal tract (GIT) and also to increase intestinal permeation.

Further, it is an object to provide a pharmaceutical composition (carrier system), taking into consideration the toxicological properties of the materials used for preparation of the composition and their ability to enhance the drug role inside the human body.

Finally, it is an object to provide a pharmaceutical composition which can advantageously be used for oral administration of drugs, in particular of protein drugs such as insulin.

This objects are achieved by a pharmaceutical emulsion composition comprising: at least one drug, at least one amino acid, at least one fatty acid and at least one surfactant, wherein the amino acid, the fatty acid and the surfactant form micelles and the drug is encapsulated into the micelles.

Preferably, the amino acid and the fatty acid form a non-covalent complex in the pharmaceutical emulsion composition. The pharmaceutical emulsion composition can be considered as a nano lipo-amino acid emulsion.

Preferably, the drug is a protein and/or a polypeptide, preferably insulin, an insulin analog and/or an insulin derivative.

It will be understood by those skilled in the art that proteins or polypeptides used in the inventive pharmaceutical emulsion composition, for example insulin derivatives, will preferably have a positive pharmaceutical effect.

Even preferred, the concentration of the drug is in a range from 5-500 IU/ml, preferably in a range from 40-100 IU/ml, most preferably 50 IU/ml.

In a preferred embodiment, the amino acid is L-arginine, L-histidine or L-lysine in form of monomers, dimers or polypeptides.

Further preferred, the fatty acid is an unsaturated fatty acid, preferably an omega-3,6 and/or 9 unsaturated fatty acid or its derivative, more preferably oleic acid, linolenic acid or linoleic acid, most preferably oleic acid.

Preferably, the surfactant is a non-ionic surfactant, preferably a fatty acid ester or an alcohol ester, most preferably is selected from the group of polyoxyethylene sorbitanes, for example from commercial available surfactants such as Spans®, Tweens®, i.e. Tween80, Cremophor, Brij®, poloxamers etc.

Even preferred, the surfactant concentration in the pharmaceutical emulsion composition is within a range from 5-80% w/w, preferably 35-75% w/w, most preferably 45-50% w/w.

In a further embodiment, the inventive pharmaceutical composition is further comprising an acidic polysaccharide or its salt, preferably sodium carboxymethyl cellulose, sodium alginate and/or algenic acid.

Preferably, the concentration of the acidic polysaccharide or its salt is within 0.01-10% w/w, preferably 0.02-0,1 % w/w, most preferably 0.03-0.05% w/w.

In preferred embodiments, the inventive pharmaceutical emulsion composition is additional comprising a buffering agent.

Preferably, the buffering agent is a TRIS-buffer, preferably 2-amino-2-hydroxymethyl-1,3-propandiol and its corresponding salts.

The objects are further achieved by the inventive pharmaceutical emulsion composition for use as a medicament.

The objects are also achieved by the inventive pharmaceutical emulsion composition for use as a medicament for oral administration.

Finally, the objects are achieved by the inventive pharmaceutical emulsion composition for use in treatment of hyperglycemia and/or diabetes.

L-arginine was selected as a preferred amino acid due to its advantageous properties. It has a favorable pharmacological effect; L-arginine has an important impact on glucose metabolism on one hand. L-arginine causes insulin release from pancreatic Beta cells. Data from three model systems support the hypothesis that L-arginine-derived nitrogen oxides (NOs) mediate insulin release stimulated by L-arginine in the presence of D-glucose and by the hypoglycemic drug tolbutamide. It is known that the L-arginine is potentiating glucose-induced insulin secretion which occurs independently of nitric oxide. It is further known that ingested arginine did not stimulate a rise in insulin concentration but it attenuates the increase in glucose when given with glucose.

On the other hand, L-arginine is useful when mixed with insulin since it prevents insulin aggregation. Thus it has an impact on insulin stabilization during the step of formulation. L-arginine is known to be one of the most widely used low molecular weight solution additives effective in suppressing aggregation, assisting refolding of aggregated proteins, and enhancing the solubility of aggregation-prone unfolded molecules in vitro.

The other preferred additive selected was oleic acid as the lipid structure due to the advantageous properties. In the art, oleic acid was found to stimulate insulin secretion in high concentration. In one study, the isolated perfused rat pancreas was utilized to investigate the effect of oleic acid on insulin secretion. In the absence of glucose, a continuous infusion of oleic acid induces a biphasic insulin release. It was demonstrated that high concentrations of oleic acid can stimulate insulin release. On the other hand, oleic acid was found to increase drug permeation through skin or mucus membranes. Oleic acid was found to increase intestinal absorption of some active materials.

The combination of L-arginine and oleic acid results in the formation of micelles, vesicle-like highly associated aggregates, and lamellar liquid crystals with large amount of water.

The presence of oleic acid in the preparation can also counteract the effect of L-arginine on glucogon secretion. Old studies were indicating that L-arginine also induce secretion of glucagon, however, the presence of circulating oleic acid as free fatty acid levels was involved in the suppression of glucagon secretion from the isolated perfused rat pancreas.

The inventive pharmaceutical composition is preferably a micro or nano lipo amino acid for protein oral delivery.

Also methods for producing the inventive pharmaceutical composition are a solution for the objects of the invention described.

It was surprisingly found that the inventive pharmaceutical emulsion composition results in a powerful hypoglycemic action upon oral administration by allowing transport of a protein drug insulin through the gastrointestinal tract with increased intestinal permeation by using only toxicological harmless substances for preparing the composition.

Further characteristics and advantages of the invention result from the detailed description of the preferred embodiments, in particular in context with the examples and the attached drawings.
Figure 1: FTIR-spectra of oleic acid, L-arginine, and a complex of oleic acid and L-arginine;

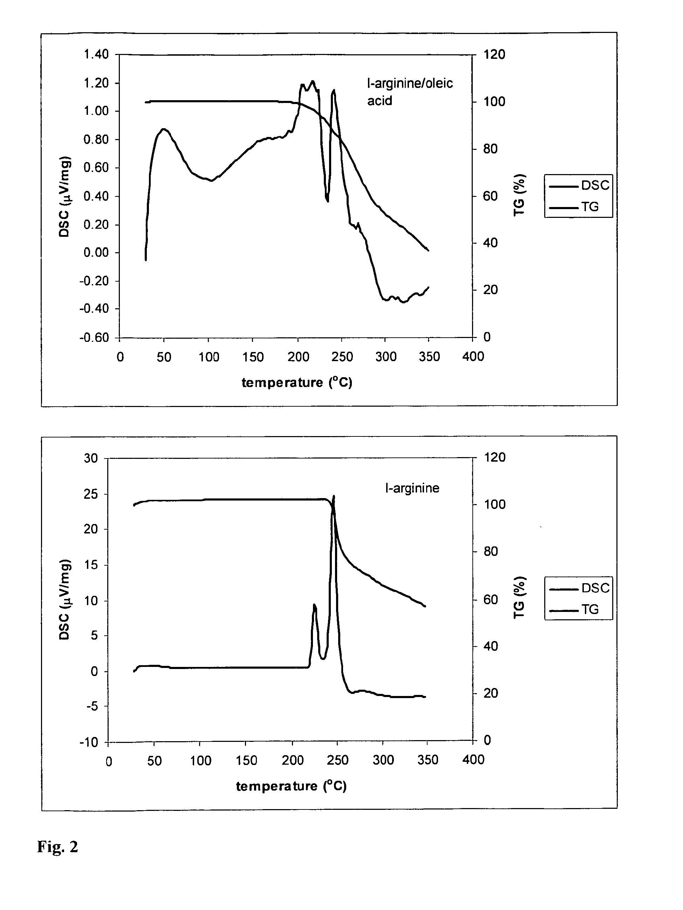

Figure 2: DSC/TGA thermograms of oleic acid, L-arginine and combination of oleic acid- L-arginine;
Figure 3: The change of viscosity of oleic acid upon the addition of 1% w/v L-arginine solution;
Figure 4: Particle size distribution of rh-insulin in aqueous solution and formulated insulin in liquid preparation;
Figure 5: HPLC chromatograms of Standard rh-insulin (25 U/ml) and Actrapid ® Injection (12.5 U/ml);
Figure 6: Percent decrease in glucose given to fasted healthy albino rabbits given oral Actrapid formulations (10 IU/kg) versus 2U/kg SC Actrapid;
Figure 7: Percent decrease in glucose level in healthy rabbits given oral insulin preparation (25 and 50 U/kg) compared to placebo.

### Examples

### Example 1

### Determination of the particle size distribution of Insulin and/or L-arginine by number as D (n, 0.5) and by intensity as D (i, 0.5) using nanosizer

The particle size distribution of two separate solutions of insulin (1 U/ml) prepared using 0.1 M HCl and L-arginine (1 mg/ml) prepared in distilled water was determined. In addition, a mixture of particles prepared by mixing insulin and L-arginine previous solutions in 1:1 v/v ratio was assessed by photon correlation spectroscopy using a Malvern Zetasizer Nano-ZS series (Malvern Instruments, UK). Collective 10 readings were performed three times on a sample at 25 °C with a detection angle of 173°. The median and standard deviations were calculated by the instrument. The viscosity was determined using a Vibro viscometer (SV-10, A&D Company, Japan) at 25°C.

Table 1 and Table 2 indicate the particle size of insulin, L-arginine and combination of insulin and L-arginine. The particle size of insulin (monomer, dimer or hexamer) is very small i.e. insulin was below 5 nm at room temperature and at 50°C for 2 hrs, however, after 24 hrs at 50°C the size could be doubled which could indicate that some insulin molecules could result in aggregation as indicated by D (i, 0.5) but the majority is still unchanged as indicated by D (n, 0.5). In addition, it was found that L-arginine forms larger particles 100-150 nm size than insulin in the aqueous solution.

The low concentration of L-arginine (1 mg/ml) could result in easily dissociation of insulin as free insulin especially when the temperature of the preparation was increased to 50°C as measured by D (n,0.5), however, this dissociation was not observed when L-arginine concentration increased to (10 mg/ml).

**Table 1: Insulin L-arginine particle size distributions by number**

| Median particle size (n, 0.5) | Room temp for 24 hr | | Stability in form of particle size at 50°C for 2 hrs | | Stability in form of particle size at 50 °C for 24 hrs | |
|---|---|---|---|---|---|---|
| | Pdl | D(n) | Pdl | D(n) | Pdl | D(n) |
| insulin (1 U/ml) | **0.291** | **4.24** | **0.519** | **3.7** | **0.327** | **3.25** |
| SD | 0.236 | 1.16 | 0.326 | 0.99 | 0.197 | 1.76 |
| L-arginine (1 mg/ml) | **0.474** | **74.8** | **0.517** | **102** | **0.31** | **93.8** |
| SD | 0.11 | 13.6 | 0.423 | 0.273 | 0.9 | 3.4 |
| L-arginine (10mg/ml) | **0.442** | **109** | **0.522** | **85.8** | **0.436** | **80.6** |
| SD | 0.164 | 1 | 0.083 | 9.56 | 0.082 | 42.3 |
| insulin 1U/ml+L-arginine 1mg/ml (1:1 volume ratio) | **0.38** | **112** | **0.709** | **3.68** | **0.209** | **3.68** |
| SD | 0.111 | 19.1 | 0.276 | 0.436 | 0.276 | 0.436 |
| insulin 1U/ml+L-arginine 10mg/ml (1:1 volume ratio) | **0.17** | **113** | **0.305** | **119** | **0.321** | **125** |
| SD | 0.039 | 3.46 | 0.011 | 4 | 0.035 | 5.51 |

| | | | | | | |
|---|---|---|---|---|---|---|
| SD: standard deviation | | | | | | |

**Table 2: Insulin L-arginine particle size distributions by intensity**

| Median particle size (n, 0.5) | Room temp for 24 hr | | Stability in form of particle size at 50 °C for 2 hrs | | Stability in form of particle size at 50°C for 24 hrs | |
|---|---|---|---|---|---|---|
| | Pdl | D(i) | Pdl | D(i) | Pdl | D(i) |
| insulin (1U/ml) | **0.291** | 3.52 | **0.519** | 4.78 | **0.327** | 8.17 |
| SD | 0.136 | 0.693 | 0.326 | 0.845 | 0.197 | 1.95 |
| L-arginine (1 mg/ml) | **0.474** | 122 | **0.517** | 115 | **0.31** | 148 |
| SD | 0.11 | 17 | 0.423 | 29.2 | 0.9 | 26.7 |
| L-arginine (10mg/ml) | **0.442** | 129 | **0.522** | 174 | **0.436** | 126 |
| SD | 0.164 | 17.2 | 0.083 | 21.6 | 0.082 | 19.5 |
| insulin IU/ml+L-arginine 1mg/ml (1:1 volume ratio) | **0.38** | 167 | **0.709** | 111 | **0.209** | 111 |
| SD | 0.111 | 35.8 | 0.276 | 21 | 0.276 | 21 |
| insulin 1U/ml+L-arginine 10mg/ml (1:1 volume ratio) | **0.17** | 170 | **0.305** | 140 | **0.321** | 146 |
| SD | 0.039 | 7.37 | 0.011 | 14.6 | 0.035 | 8.96 |

| | | | | | | |
|---|---|---|---|---|---|---|
| SD: standard deviation | | | | | | |

### Example 2

### FTIR oleic acid-L-arginine complex

A sample of 200 mg of L-arginine was dispersed in few drops (about 5) of distilled water and mixed with 0.5 ml oleic acid to form slurry. The slurry was triturated with 2 gram KBr. The sample was scanned using FTIR spectrometer. A blank sample was prepared containing KBr and the added water and used as FTIR background. About 5-10 mgs of L-arginine and oleic acid samples were added separately to 200 mg KBr were also measured by FTIR.

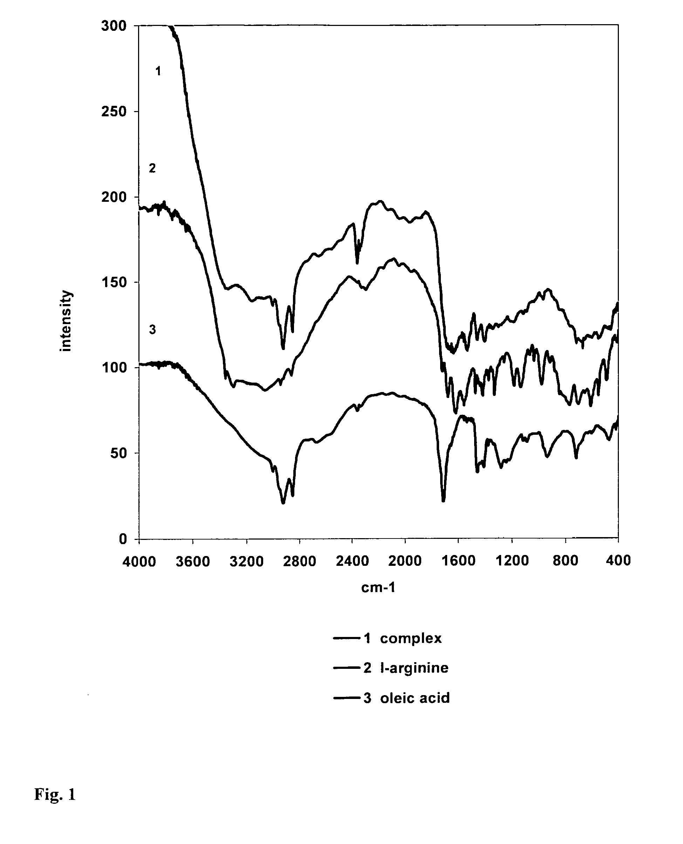

As indicated by the FTIR in figure 1, the amino groups of L-arginine could react with carboxylate functional groups in oleic acid (1700-1720 nm), which may suggest the formation of lipo amino nanoparticle delivery system

### Example 3

### DSC/TGA of oleic acid/L-arginine complex

10 mg of L-arginine and 100 mg oleic acid mixture were put in an open DSC cup scanned by a previously calibrated simultaneous thermal analyzer (STA). Another 10 mg L-arginine sample was scanned without oleic acid. The scanning rate was 10 °C/minute. It was obvious that L-arginine reacts with oleic acid at temperature of 50-150 °C. Also, the degradation pattern at 220 °C for L-arginine in the presence of oleic acid was different which indicates the formation of some sort of interaction at the beginning.

### Example 4

### Viscosity change upon the addition of L-arginine to oleic acid

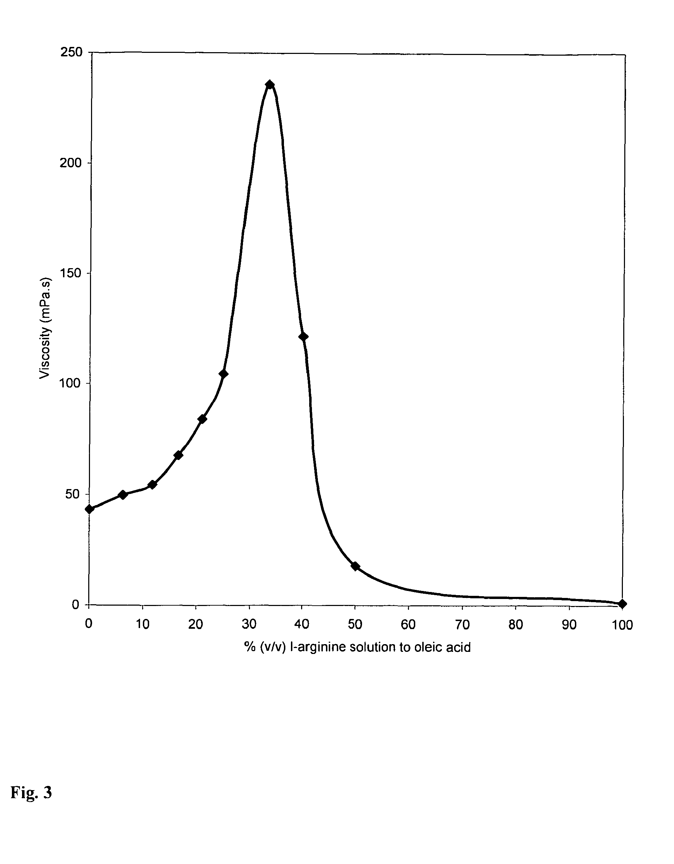

L-arginine (1%) was mixed with different proportion with oleic acid with the aid of magnetic stirrer. The produced emulsion was measured using Vibro viscometer at each proportion. Fig. 3 indicates the increase in viscosity upon the addition of L-arginine solution to oleic acid until reaching 30% volume of aquesous solution then the viscosity was dropped suddenly which could indicate the phase inversion of the emulsion.

### Example 5

### Particle size distribution of oleic acid/L-arginine mixture and the possibility to be used for insulin oral delivery

The particle size was measured using Malvern Zetasizer for a combination of oleic acid/L-arginine (10 mg/ml) mixed in a 10: 1 volume ratio as indicated in Table 3. The resulted particle size was large reaching the micro-level which indicates the need of a surfactant to decrease the particle size in order to be converted into nanoparticles suitable for oral insulin delivery.

**Table 3: Particle size distribution by number and intensity**

| | oleic acid/L-arginine | (10 mg/ml) | (10:1 volume ratio) |
|---|---|---|---|
| | PdI | D(n) | D(i) |
| Median | 0.649 | 1020 | 1070 |
| SD | 0.385 | 496 | 515 |

### Example 6

### Preparation of the Pharmaceutical emulsion composition

20 gram of oleic acid and another 20 gram Tween 80 ® were mixed using a magnetic stirrer for 10 minutes (solution 1). 200 mg rh-insulin was dissolved in 2ml 0.1 M HCl. In another test tube 100 mg L-arginine was dissolved in 1 ml. L-arginine solution was added drop wise to the 2 ml insulin solution until pH become 7-7.5. To the insulin/L-arginine solution 1 ml sodium alginate (1% w/v) solution was added and mixed gently (solution 2). Then, 1 ml of solution 2 was added dropwise with rapid mixing to 10 ml of solution 1.

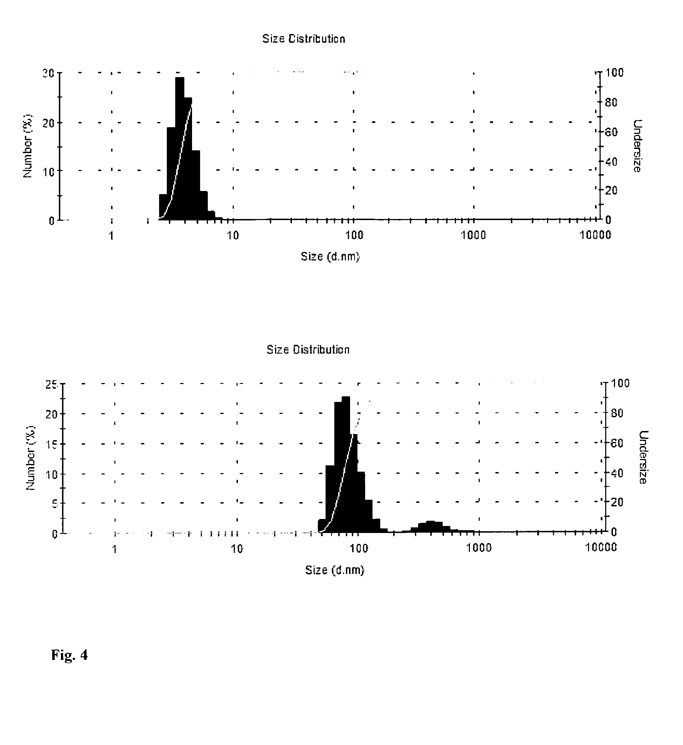

The particle size of the preparation was determined for insulin prior to formulation and after formulation as depicted in Figure 4. The figure indicated the capability of conversion of insulin to nanoparticles using surfactants such as Tween 80 ®.

### Example 7

### Preparation of composition using commercial Actapid penfill (fast acting) insulin injection and testing on fasted normal rabbits

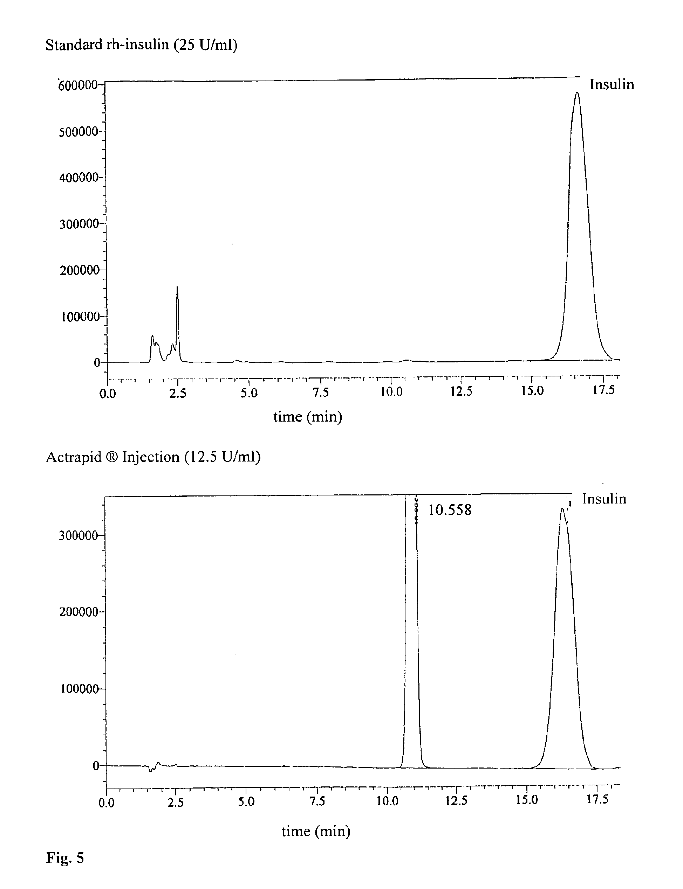

Prior to mixing L-arginine with Actrapid penfill the insulin peak was determined for in Actrapid injection in comparison to insulin reference solution using HPLC method (USP) as shown in Fig. 5

20 gram of oleic acid and another 20 gram Tween 80 were mixed using a magnetic stirrer for 10 minutes (solution 1). 0.5 ml Actrapid penfill (100 U/ml insulin) was add with 0.5 ml alginate (1% w/v) solution and mixed gently (solution 2). 50 mg L-arginine was added dissolved with the Actrapid/alginate solution. Then, 1 ml of the produced solution was dropped in 10 ml of oleic acid/Tween 80 mixture and mixed vigorously.

### Example 8

### In vivo response of oral Actrapid insulin (formulated) given to healthy rabbits

The Actrapid preparation of example 7 was given orally to healthy albina rabbits. 12 rabbits aged about 18-20 weeks and weighing 1.0-1.53 kg were acclimatized for two weeks. The animals were fed standard rabbit chow. The rabbits were divided into four groups (each 3) and each group got different treatment. The rabbits were fasted for 18 hr prior to the test. Group 1 rabbits were given oral nanoparticle preparation (oral formulated Actrapid insulin 10 IU/kg). Group 2 rabbits were given Actrapid ® subcutaneous injection (2 IU/kg). Group3 rabbits were given Actrapid ® injection as an oral solution (non formulated) in a dose of 10 IU/kg. Group 4 were given oral placebo preparation. Glucose level was monitored using Glucometer at different time intervals.

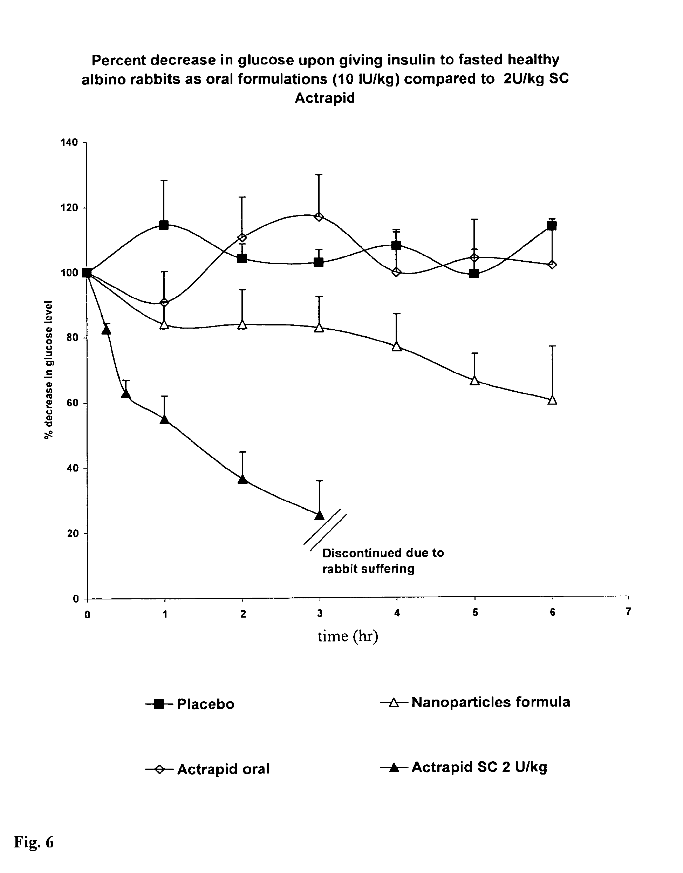

Fig. 6 indicated the decrease in blood glucose level after the administration of oral Actrapid formulated in Lipo nanoparticles in comparison to placebo or oral Actrapid injection (non-formulated).

### Example 9

### In vivo pharmacological response of oral rh-insulin (raw powder)

Preparation of insulin pharmaceutical emulsion composition: Insulin was dissolved gently in 2 ml 0.1 M HCl, in another test tube 50 mg L-arginine was dissolved in 1 ml distilled water. The L-arginine solution was added drop wise with gentle stirring to insulin solution. To the prepared insulin powder/L-arginine solution, 1 ml sodium alginate solution (1% w/v) was added with gentle stirring. In other beaker, a liquid mixture of Tween 80 and oleic acid (1:1 w/w) was mixed together for 30 minutes. Then 1 ml solution of insulin/L-arginine/sodium alginate was added drop wise with mixing to 10 ml of Tween 80/oleic acid mixture.

Animal study: 15 fasted healthy albino rabbits were classified into 3 groups (5 in each) and given the hollowing preparations:
a) Oral insulin formulated in the pharmaceutical emulsion composition was given in a dose of 25 and 50 IU/kg
b) Oral preparation of the pharmaceutical emulsion composition without insulin (placebo) used for comparison.
c) Oral water (control) used for comparison

Glucose level was monitored using glucometer at different time interval.

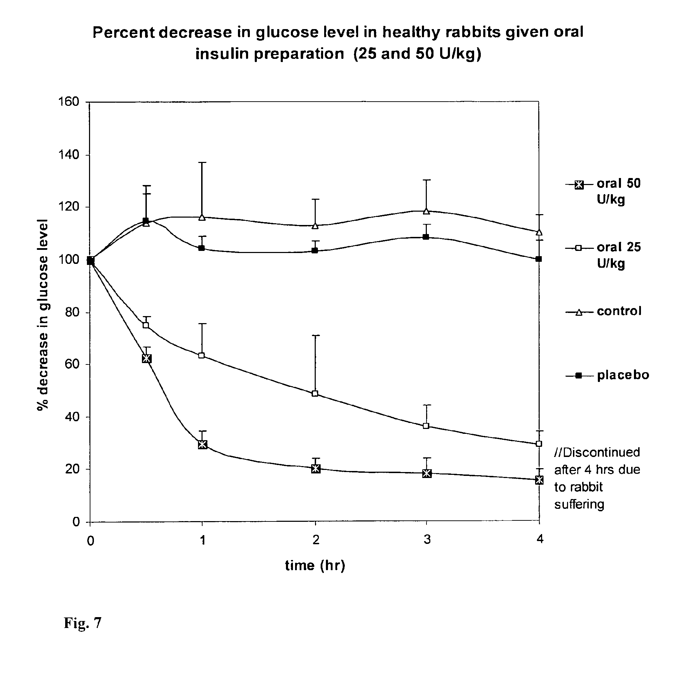

Fig. 7 showed a tremendous decrease in glucose level after oral administration insulin formulated as nano-lipo amino acid (pharmaceutical emulsion composition) in comparison to control or placebo preparation. The decrease in glucose level was found to be dependent on insulin dose i.e. the decrease in glucose level upon giving 50 IU/kg was higher than that of 25 IU/kg. This could obviously indicate insulin pharmacological response; hence the decrease glucose level is an indicator for the absorption of insulin through the gastrointestinal tract as intact molecules.

The features disclosed in the foregoing description, in the claims and/or in the accompanying drawings may, both separately and in any combination thereof, be material for realizing the invention in diverse forms thereof.

## Claims

1. Pharmaceutical emulsion composition comprising: at least one drug, at least one amino acid, at least one fatty acid and at least one surfactant, wherein the amino acid, the fatty acid and the surfactant form micelles and the drug is encapsulated into the micelles.

2. Pharmaceutical emulsion composition according to claim 1, wherein the drug is a protein and/or a polypeptide, preferably insulin, an insulin analog and/or an insulin derivative.

3. Pharmaceutical emulsion composition according to claim 1 or 2, wherein the concentration of the drug is in a range from 5-500 IU/ml, preferably in a range from 40-100 IU/ml, most preferably 50 IU/ml.

4. Pharmaceutical emulsion composition according to any of the preceding claims, wherein the amino acid is L-arginine, L-histidine or L-lysine in form of monomers, dimers or polypeptides, preferably L-arginine.

5. Pharmaceutical emulsion composition according to any of the preceding claims, wherein the fatty acid is an unsaturated fatty acid, preferably an Omega-3,6 and/or 9 unsaturated fatty acid or its derivative, more preferably oleic acid, linolenic acid or linoleic acid, most preferably oleic acid.

6. Pharmaceutical emulsion composition according to any of the preceding claims, wherein the surfactant is a non-ionic surfactant, preferably a fatty acid ester or an alcohol ester, most preferably is selected from the group of polyoxyethylene sorbitans.

7. Pharmaceutical emulsion composition according to any of the preceding claims, wherein the surfactant concentration in the pharmaceutical emulsion composition is within a range from 5-80% w/w, preferably 35-75% w/w, most preferably 45-50% w/w.

8. Pharmaceutical emulsion composition according to any of the preceding claims, further comprising an acidic polysaccharide or its salt, preferably sodium carboxymethyl cellulose, sodium, alginate and/or algenic acid.

9. Pharmaceutical emulsion composition according to claim 8, wherein the concentration of the acidic polysaccharide or its salt is within 0.01-10% w/w, preferably 0.02-0.1% w/w, most preferably 0.03-0.05% w/w.

10. Pharmaceutical emulsion composition according to any of the preceding claims additional comprising a buffering agent.

11. Pharmaceutical emulsion composition according to any of the preceding claims, wherein the buffering agent is a TRIS-buffer, preferably 2-amino-2-hydroxymethyl-1,3-propandiol and its corresponding salts.

12. Pharmaceutical emulsion composition according to any of the preceding claims for use as a medicament.

13. Pharmaceutical emulsion composition according to any of the preceding claims for use as a medicament for oral administration.

14. Pharmaceutical emulsion composition according to any of the preceding claims for use in treatment of hyperglycemia and/or diabetes.
